# FASCICULE DE BREVET EUROPEEN

(11) **EP 1 687 276 B1**
(45) Date de publication et mention de la délivrance du brevet: **14.10.2009**
(21) Numéro de dépôt: 04805450.6
(22) Date de dépôt: 15.11.2004
(51) Int. Cl.: C07D 215/18, C07D 215/38

(54) **NOUVEAU PROCEDE DE PREPARATION DE QUINOLEINES 3-FLUOREES**
NEUES VERFAHREN ZUR HERSTELLUNG VON 3-FLUORIERTEN CHINOLINEN
NOVEL METHOD OF PREPARING 3-FLUORINATED QUINOLINES

(30) Priorité: 17.11.2003 FR 0313384
(43) Date de publication de la demande: 09.08.2006
(73) Titulaire: NOVEXEL, 93230 Romainville (FR)
(72) Inventeur: EL-AHMAD, Youssef, 94000 Creteil (FR); LECONTE, Jean-Pierre, 91800 Brunoy (FR); MALPART, Jol, 45160 Olivet (FR); MIGNANI, Serge, 92290 Chatenay Malabry (FR); MUTTI, Stéphane, 94170 Le Perreux sur Marne (FR); TABART, Michel, 91290 La Norville (FR)
(74) Mandataire: Hirsch & Associés
(86) Numéro de dépôt international: PCT/FR2004/002910
(87) Numéro de publication internationale: WO 2005/049575

(56) Documents cités:
- ARTHUR FROE AND G.F. HAWKINS: "The preparation of Heterocyclic Fluorine Compounds by the Schiemann Reaction. II The Monofluoroquinolines" J. AM. CHEM. SOC., vol. 71, 1949, pages 1785-6, XP002288033

## Description

L'invention a pour objet un nouveau procédé de préparation de quinoléines 3-fluorées de formule générale (I) : 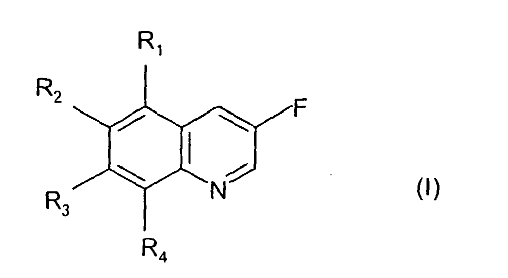 dans laquelle R₁, R₂, R₃ et R₄, identiques ou différents, représentant :
- un atome d'hydrogène ou de fluor ;
- un radical alkyle linéaire, ramifié ou cyclique, éventuellement substitué par un à trois atomes de fluor, par un groupement OR₅ dans lequel R₅ représente un radical alkyle linéaire ou ramifié, un atome d'hydrogène
ou un groupement protecteur de radical hydroxy, ou par un groupement NR'R" dans lequel R' et R", indentiques ou différents, représentent un radical alkyle linéaire ou ramifié, un atome d'hydrogène ou un groupement protecteur du radical amino ;
- un groupement OR₆ dans lequel R₆ représente un atome d'hydrogène, un groupement protecteur du phénol ou un radical alkyle linéaire ou ramifié, éventuellement substitué par un à trois atomes de fluor, par OR₅ ou par NR'R" tels que définis ci-dessus ;
- un groupement NR'₁R"₁ dans lequel R'₁ et R"₁ ont les valeurs de R' et R" ou représentent un radical alkyle linéaire ou ramifié substitué par un à trois atomes de fluor, par un groupement OR₅ tel que défini ci-dessus ou par un groupement NR'R" tel que défini ci-dessus ; un groupement CO₂Rₐ, dans lequel Rₐ représente un atome d'hydrogène, un radical alkyle linéaire ou ramifié ou un groupement protecteur du radical carboxy ;
- un radical phényle ou un radical hétéroaryle, éventuellement substitué par un ou plusieurs des substituants mentionnés plus haut.

Dans la formule générale ci-dessus, il est entendu que les radicaux alkyle contiennent 1 à 10 atomes de carbone en chaîne droite ou ramifiée et que les radicaux cycloalcoyle contiennent 3 à 6 atomes de carbone.

Lorsque les composés portent un substituant hétéroaryle, ce dernier contient 5 à 10 chaînons et peut être mono ou bicyclique et choisi (à titre non limitatif) parmi thiényle, furyle, pyrrolyle, imidazolyle, thiazolyle, oxazolyle, thiadiazolyle, oxadiazolyle, tétrazolyle, pyridyle, pyridazinyle, pyrazinyle, pyrimidinyle, indolyle, benzothiényle, benzofuranyle, indazolyle, benzothiazolyle, naphtyridinyle, quinolyle, isoquinolyle, cinnolyle, quinazolyle, quinoxalyle, benzoxazolyle, benzimidazolyle pouvant être éventuellement substitués par les substituants énoncés ci-dessus.

Arthur FROE et G.F. HAWKINS, J. Am. Chem. Soc., vol.71,1949, pages 1785-6,décrivent la préparation de monofluoro de quinoléines non substituées par ailleurs, au départ des amino quinoléines correspondantes, par la réaction de Schiemann.

Selon l'invention, les produits de formule générale (I) sont obtenus par un procédé **caractérisé en ce que** l'on soumet un composé de formule générale (IV) : 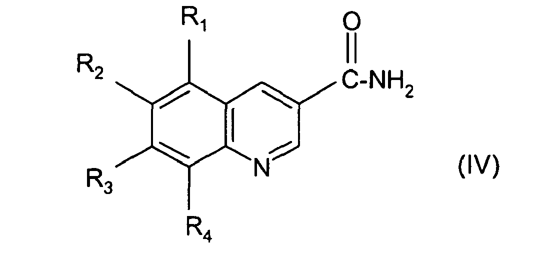 dans laquelle R₁, R₂, R₃ et R₄ conservent les définitions précitées, à une dégradation d'Hofmann, pour obtenir un composé de formule générale (III) 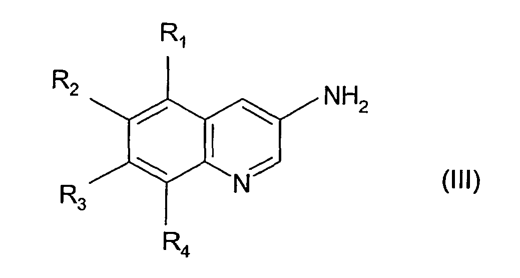 dans laquelle R₁, R₂, R₃ et R₄ conservent les définitions précitées, que l'on traite dans des conditions susceptibles de former le sel de diazonium de formule générale (II) 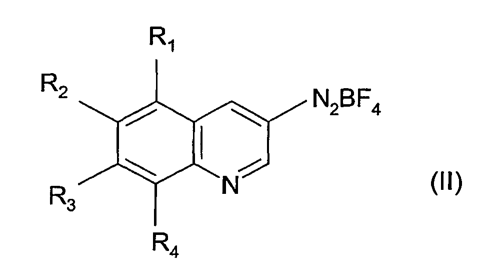 dans laquelle R₁, R₂, R₃ et R₄ conservent les définitions précitées, que l'on chauffe au sein d'un solvant organique inerte, à une température comprise entre 35 et 120°C.

La dégradation d'Hofmann est effectuée d'hydroxyde de sodium et de brome, ainsi que de pyridine, dans l'eau, à une température d'environ 60°C.

Les conditions de préparation du sel de diazonium consistant par exemple à opérer en présence d'un sel alcalin ou d'un ester de l'acide nitreux, notamment de nitrite de sodium ou de potassium ou de nitrite de tert-butyle ou d'isobutyle, et d'acide fluoroborique ou en présence de complexe trifluorure de bore-éther éthylique, au sein d'un solvant approprié, notamment le THF l'eau ou un alcool, à une température comprise entre +15 et +20°C.

Pour transformer le composé de formule (II) en composé de formule (I) on opère de préférence au sein d'un solvant tel que le toluène, le xylène, l'heptane, l'hexane, un solvant fluoré tel que le perfluorohexane, ou encore un solvant chloré tel que le mono ou le dichlorobenzène, le chlorobutane ou le chlorure de méthylène.

La température de chauffage est de préférence comprise entre 60 et 100°C et est, bien entendu, fonction du solvant utilisé.

L'invention a également pour objet un procédé selon ce qui précède, **caractérisé en ce que** le composé de formule générale (IV) est obtenu en soumettant un composé de formule (V) 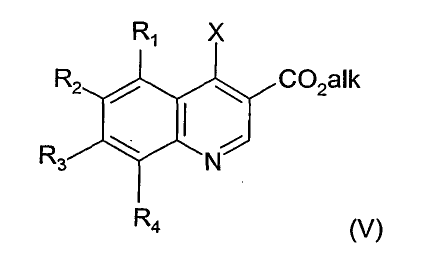 dans laquelle R₁, R₂, R₃ et R₄ conservent les définitions précitées, X représente un atome de chlore ou de brome et alk représente un radical alkyle renfermant de 1 à 6 atomes de carbone, à l'action d'un agent d'hydrogénolyse, puis à celle de l'ammoniaque pour obtenir successivement le composé de formule (VI) 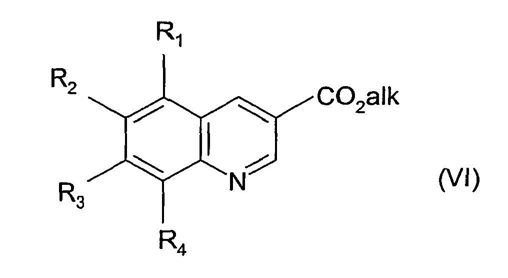 que l'on isole ou non, puis le composé de formule (IV).

Selon l'invention, les deux réactions ci-dessus peuvent être conduites dans l'ordre inverse, le composé formé intermédiairement, et que l'on isole ou non, étant alors le composé de formule (VII)

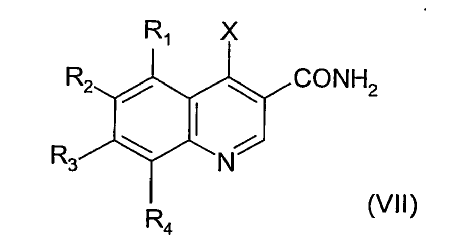

La réaction d'hydrogénolyse est effectuée dans un alcool, notamment l'éthanol ou le méthanol, en présence de triéthylamine et d'un catalyseur tel que le palladium sur charbon, en faisant barboter de l'hydrogène dans le milieu réactionnel.

On peut encore opérer au sein du diméthylformamide, en présence de formiate de sodium et de tétrakis (triphénylphosphine) palladium.

L'invention a également pour objet un procédé selon ce qui précède, **caractérisé en ce que** le composé de formule (V) est obtenu en soumettant un composé de formule (VIII) 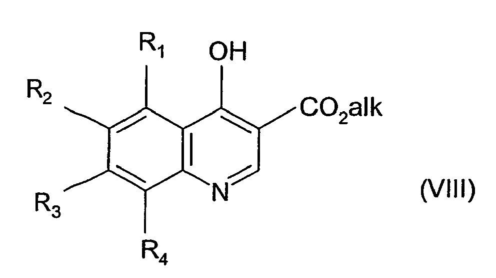 dans laquelle R₁, R₂, R₃ et R₄ et alk conservent les définitions précitées, à l'action de l'oxychlorure ou l'oxybromure de phosphore.

On opère de préférence sans solvant, à une température de l'ordre de 100°C.

L'invention a également pour objet un procédé selon ce qui précède, **caractérisé en ce que** le composé de formule (IV) est obtenu en traitant un composé de formule (VIII), telle que définie précédemment, par une base, pour obtenir un acide correspondant de formule (IX) 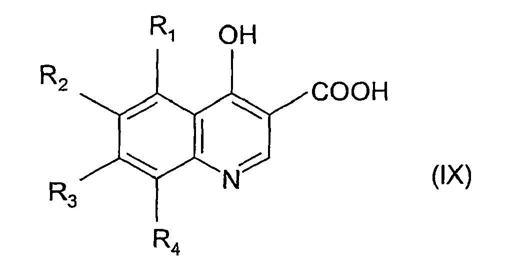 dans laquelle R₁, R₂, R₃, et R₄ conservent les définitions précitées, que l'on soumet à l'action de l'oxychlorure ou de l'oxybromure de phosphore, pour obtenir un composé de formule (X) 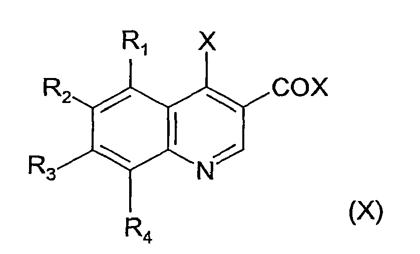 dans laquelle R₁, R₂, R₃, R₄ et X conservent les définitions précitées, lequel est soumis à l'action de l'ammoniac pour obtenir un composé de formule (VII) telle que définie précédemment, que l'on soumet à l'action d'un agent d'hydrogénolyse.

La réaction de saponification est réalisée dans des conditions classiques connues de l'homme du métier, notamment par action de l'hydroxyde de sodium ou de potassium en milieu acqueux ou reflux.

L'action de l'oxychlorure ou de l'oxybromure de phosphore est de préférence effectuée au reflux de ce dernier, sans solvant.

L'agent d'hydrogénolyse est l'un de ceux qui ont été cités plus haut.

L'invention a également pour objet un procédé selon ce qui précède, **caractérisé en ce que** le composé de formule (IV) est obtenu en chauffant un composé de formule 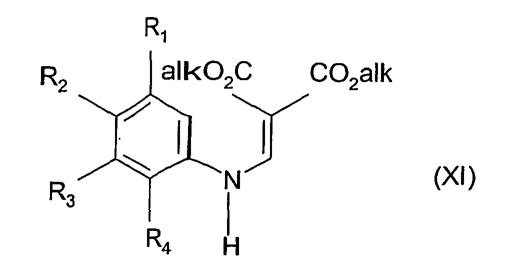 dans laquelle R₁, R₂, R₃, R₄ et alk conservent les définitions précitées, en présence de pentoxyde de phosphore, pour obtenir un composé de formule (IX) telle que définie précédemment, puis poursuit la synthèse comme décrit précédemment.

La réaction du pentoxyde de phosphore sur le composé de formule (XI) est effectuée de préférence au sein d'un solvant tel que le nitrobenzène à une température de l'ordre de 120-130°C.

Le composé de formule (V) peut encore être obtenu en soumettant un composé de formule (XI) telle que définie précédemment à l'action de l'oxychlorure ou de l'oxybromure de phosphore.

On opère de préférence sans solvant, à une température de l'ordre de 100°C, au départ d'un composé de formule (XI) dans lequel les groupements sensibles au niveau de R₁, R₂, R₃, R₄ sont préalablement protégés.

Le composé de formule (VIII) peut être obtenu en chauffant un composé de formule (XI) telle que définie précédemment, au sein d'un solvant à haut point d'ébullition.

La cyclisation du composé de formule (XI) est de préférence réalisée au sein du diphényléther, au reflux ou à une température voisine du reflux de ce dernier.

Le composé de formulé générale (XI) est obtenu en faisant réagir un composé de formule générale (XII) 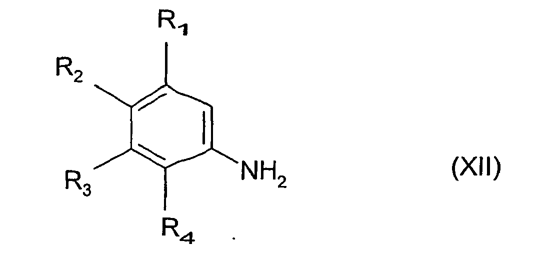 dans laquelle R₁, R₂, R₃ et R₄ conservent les définitions précitées, avec un composé de formule générale (XIII) 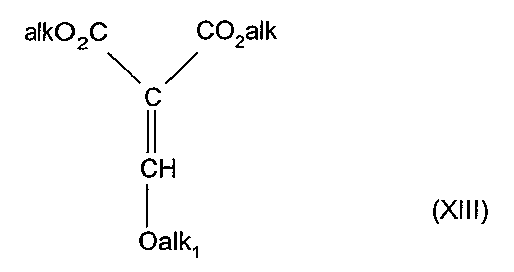 dans laquelle alk conserve la définition précitée et alk₁ représente un radical alkyle linéaire ou ramifié, identique ou différent de alk.

Dans des conditions préférentielles, la réaction est effectuée sans l'addition d'un solvant, pour chauffage à une température comprise entre 80 et 120°C.

Selon les définitions de R₁, R₂, R₃ et R₄, il peut être souhaitable, voire nécessaire, selon le type de réaction impliqué, de mettre en oeuvre des composés dans lesquels les substituants sont protégés. Il s'agit en particulier des substituants alkyles substitué par OR₅ ou par NR'R", OR₆, NR'₁R"₁ et CO₂Rₐ.

L'invention a notamment pour objet un procédé tel que défini précédemment, **caractérisé en ce que** l'on met en oeuvre des composés dans lesquels les éventuels substituants sensibles sont protégés, lors de la préparation des composés de formule (X) à partir des composés de formule (IX) ainsi que lors de la dégradation d'Hofmann conduisant aux composés de formule (III) à partir des composés de formule (IV). Selon l'invention, les composés impliqués sont soit protégés depuis le début de la synthèse soit juste avant la réalisation de l'étape critique.

Les groupements protecteurs que l'on peut utiliser ainsi que leur mise en oeuvre sont connus de l'homme du métier et décrits, par exemple, par T.W. Greene et P.G.M.Nuts dans l'ouvrage « Protective Groups in Organic Synthesis » (John Wiley & Sons, inc.).

L'invention a notamment pour objet un procédé selon ce qui précède, **caractérisé en ce que** l'on met en oeuvre des composés dans lesquels R₁, R₂, R₃ et R₄, identiques ou différents, représentent un atome d'hydrogène, un atome de fluor, un radical alkyle linéaire ou ramifié éventuellement substitué tel que défini précédemment, ou un radical OR₆ tel que défini précédemment.

L'invention a plus particulièrement pour objet un procédé **caractérisé en ce que** l'on met en oeuvre des composés dans lesquels R₁, R₂, R₃ et R₄ identiques ou différents, représentent un atome d'hydrogène, un atome de fluor ou un radical OR₆ tel que défini précédemment et notamment un radical alkoxy.

L'invention a enfin pour objet les composés de formule (II) tels que définis plus haut.

Les composés de formule (I) sont des composés intermédiaires utiles notamment dans la préparation des composés possédant une activité antibactériennes, décrits par exemple dans les demandes WO 02/72572 et WO 02/40474.

Les exemples suivants illustrent l'invention sans toutefois la limiter.

### Exemple 1 : Préparation de la 3-fluoro-6-methoxyquinoléine :

1007g de 6-methoxy-3-quinolinediazonium fluoroborate en suspension dans 9 1 de toluène sont portés à 60°C en 85 minutes. Un dégagement gazeux est observé à 60°C. Le milieu réactionnel est ensuite chauffé progressivement pendant 90 minutes à 70-72°C. Après un maintien 90 minutes à 72°C, le milieu est ensuite porté progressivement jusqu'à 85°C. Après refroidissement et agitation pendant une nuit, 4 l d'eau glacée sont additionnés sur la suspension. Après avoir agité 15 minutes, 2,5 l d'acétate d'éthyle sont additionnés. Après avoir agité 45 minutes, le pH est ajusté à pH=7-7,5 par addition de soude à 47% (250 ml). Le milieu est agité 30 minutes puis décanté 1 heure. La phase aqueuse inférieure est ré-extraite à l'acétate d'éthyle. Les phases organiques sont rassemblées et lavées à l'eau. La solution de produit brut est filtrée puis concentrée sous pression réduite pour donner 655g de 3-fluoro-6-methoxyquinoléine brute. Le produit brut est distillé sous pression réduite. Les fractions de distillation (bp 103-110°C sous 1 mbar) contenant le produit attendu sont rassemblées. On obtient ainsi 498,9 g de 3-fluoro-6-methoxyquinoléine (76%) sous la forme d'un solide blanc fondant à 51-53°C.
Microanalyse : C₁₀H₈FNO Calculé C 67.79 ; H 4.55 ; F 10.72 ; N 7.91 ; O 9.03 Trouvé C 67.98 ; H 4.54 ; N 7.97
Spectre de R.M.N. : ¹H (300 MHz, (CD₃)₂SO d6, δ en ppm) : 3,92 (s : 3H) ; 7,40 (mt : 2H) ; 7.97 (d, J = 10 Hz : 1H) ; 8,13 (dd, J = 10 et 3 Hz : 1H) ; 8.76 (d, J = 3 Hz : 1H).

| Spectre de Masse : | | |
|---|---|---|
| m=177 | | |
| EI | m/z=177 | m⁺ pic de base |
| | m/z=134 | [M - COCH₃]⁺ |
| DCI | m/z= 178 | MH⁺ pic de base |

### 3-diazoniumfluoroborate-6-méthoxyquinoléine:

10 g de 3-amino-6-méthoxyquinoléine sont mis en suspension dans 50 ml de THF et agités 15 minutes à 20 °C, avant d'être refroidis à -15°C. 11,6 g de BF₃-éthérate sont alors additionnés. La température de la masse réactionnelle est ramenée à -15°C. Après 15 minutes à cette température, 7,5 g de nitrite de ter-butyle à 90% en solution dans 25 ml de THF sont additionnés en 10 minutes. La suspension est agitée 1 heure à -15°C, avant d'être portée à + 15°C sur une période d'une heure. Le précipité est filtré, lavé par de l'hexane puis séché à 15-20°C sous pression constante jusqu'à poids constant. On obtient 14.8 g (94.3%) de 3-diazoniumfluoroborate-6-méthoxy-quinoléine sous forme d'un solide jaune.
Température de décomposition : 82°C

### 3-amino-6-méthoxyquinoléine :

A 341 kg d'eau, sont ajoutés 104 kg d'une solution d'hydroxyde de sodium à 32%. La solution est refroidie à 0°C et 22,0 kg de brome sont introduits en 1,5 heures en maintenant la température à 0°C. La solution est agitée à cette température 1 heure, puis on introduit 409 kg de pyridine en 3 heures à 0°C. 26,5 kg de 6-méthoxy quinoléine-3-carboxamide sont alors ajoutés en 50 minutes à 0°C. Le milieu réactionnel est maintenu à cette température pendant 2 heures puis progressivement chauffé à 60°C en 1 heure. Après 6 heures de maintien à 60°C, la masse réactionnelle est refroidie à 20°C et décantée. La phase aqueuse (172 kg) est lavée avec de la pyridine (60 litres). Les phases organiques sont rassemblées (820 kg) et concentrées à sec sous pression réduite (100-150 mbar) à 84°C maximum. Le résidu est alors repris avec 425 1 d'eau et 43 1 d'éthanol. La suspension obtenue est chauffée au reflux pendant 1 heure puis refroidie. Le produit commence à précipiter à une température de 65°C. Le milieu est ensuite refroidi à 0-5°C, puis maintenu a cette température 2 heures. Le précipité est filtré, lavé par de l'eau froide, puis séché à 50-55°C sous pression réduite. On obtient 16,5 kg (72.3%) de 3-amino-6-méthoxyquinoléine sous forme d'un solide marron (titre HPLC = 99,1%), ayant un point de fusion de 108-110°C.
Un deuxième jet de 3,7 kg (13,1%) est obtenu à partir des liqueurs-mères (titre HPLC = 98%).

Spectre de R.M.N. : ¹H (300 MHz, (CD₃)₂SO d6, δ en ppm) : 3,84 (s : 3H) ; 5,65 (s : 2H) ; 6.97 (dd, J = 9 et 3 Hz : 1H) ; 7,01 (d, J = 3 Hz : 1H) ; 7,08 (d, J = 2,5 Hz : 1H) ; 7.67 (d, J = 9 Hz : 1H) ; 8.29 (d, J = 2,5 Hz : 1H).
Spectre IR : 422933 (KBr)
3454; 3312; 3204; 1630; 1619; 1607; 1504; 1383; 1251; 1239; 1216; 1167; 1027; 872; 827; 627 et 479 cm⁻¹

| | | |
|---|---|---|
| Spectre de masse : | 210129 , m=174 | |
| EI m/z=174 | M⁺· pic de base | m/z=131 [M - COCH₃]^{+·} |

### 6-méthoxy-quinoléine-3-carboxamide :

A 49,2 kg de 4-chloro-3-ethoxycarbonyl-6-methoxyquinoline sont additionnés 380 1 d'éthanol. La suspension est chauffée à 45°C pendant 30 minutes puis refroidie à 20°C. 18,65 kg de triéthylamine sont ajoutés sous azote, puis 1,91 kg de palladium sous charbon à 5% (à 60% d'humidité). On fait passer un courant d'hydrogène sous 0,5- 0,8 bar à 33°C pendant 48 heures. A ce moment, un contrôle HPLC¹ montre que la réaction est complète. Le réacteur est alors purgé à l'azote puis le milieu réactionnel est filtré pour éliminer le catalyseur. Le filtre est alors rincé à l'éthanol. Le filtrat est coulé sur 750 kg d'une solution aqueuse d'ammoniaque. Le milieu réactionnel est alors agité à 25°C sur une période de 4 jours. L'éthanol est ensuite éliminé par distillation sous pression réduite à une température ne dépassant pas 40-45°C. La suspension ainsi obtenue est refroidie à 0-5°C et agitée 3 heures à cette température. Le précipité est filtré, lavé par de l'eau froide puis séché à 60-65°C sous pression réduite jusqu'à poids constant. On obtient 26.5 kg (71%) de 6-méthoxy-quinoléine-3-carboxamide sous forme d'un solide blanc fondant à 93,7-95,7°C (HPLC_{%NIS}= 98,3%).

Spectre de R.M.N. : ¹H (300 MHz, (CD₃)₂SO d6, δ en ppm) : 3,94 (s : 3H) ; 7,45 (d, J = 3 Hz : 1H) ; 7,52 (dd, J = 9 et 3 Hz : 1H) ; 7,67 (s large : 1H) ; 8,00 (d, J = 9 Hz : 1H) ; 8,29 (s large : 1H) ; 8,74 (d, J = 2 Hz : 1H) ; 9,15 (d, J = 2 Hz : 1H).
Spectre IR : 420609 (KBr)
3408; 3330; 3211; 1697; 1626; 1511; 1386; 1321; 1240; 1023; 935; 826 et 693 cm⁻¹

| | | | |
|---|---|---|---|
| Spectre de masse : | 206132, | m=202 | |
| | EI | m/z=202 | M⁺· pic de base |
| | m/z=186 | [M -NH₂]⁺· | |
| | m/z=158 | [186 - CO]⁺· | |

### 4-chloro-3-ethoxycarbonyl-6-méthoxy-quinoléine :

A 50 g de 2-[(4-méthoxyphénylamino)-méthylène]-malonate de diéthyle sont additionnés à 25°C, 132 g de chlorure de phosphoryle. Le milieu réactionnel est agité 15 minutes à cette température, chauffé à 95-100°C en 45 minutes, puis maintenu à cette température 4 heures. L'excès de chlorure de phosphoryle est ensuite éliminé par chauffage à 125°C pendant environ 2 heures. Le mélange est alors refroidi à 25°C et 125 ml de dichlorométhane sont ajoutés. Le milieu est ensuite agité à 25°C pendant 1 heure, puis coulé en 30 minutes sur 900 ml d'eau, en maintenant la température inférieure à 30°C. Le pH est alors ajusté à 7,5-8 par addition de 172 g d'une solution d'hydroxyde de sodium à 47%, en maintenant la température à 20-25°C. Les 2 phases sont séparées et la phase aqueuse est extraite au dichlorométhane. Les phases organiques sont rassemblées et lavées à l'eau. La phase dichlorométhane est concentrée de moitié et 190 ml d'éthanol sont additionnés. La concentration est poursuivie jusqu'à ce que la température de la masse réactionnelle atteigne 82°C et la température vapeur 78°C. On refroidit à 0-5°C puis maintient 2 heures à cette température. Le précipité est filtré, lavé par de l'éthanol froid puis séché à 50°C sous pression réduite. On obtient 27,7 g (61%) de 4-chloro-3-ethoxycarbonyl-6-méthoxy-quinoléine sous forme d'un solide jaune fondant à 93,7-95,7°C.
Titre (HPLC) : 98,0 %

### 2-[(4-méthoxyphénylamino)-méthylène]-malonate de diéthyle :

A 6,25 kg d'éthoxy-méthylène malonate de diéthyle à 14°C sont additionnés 3,5 kg de p-anisidine en 85 minutes sans refroidissement de la masse réactionnelle. En fin d'addition, la température a atteint 59°C. On maintient à 59°C 30 minutes, puis le milieu réactionnel est chauffé à 90-95°C et maintenu à cette température 1 heure. L'éthanol formé est alors éliminé par distillation à pression atmosphérique puis sous 250 mbars. Après refroidissement à 45°C, 8,4 kg de 2-[(4-méthoxyphénylamino)-méthylène]-malonate de diéthyle sont récupérés sous forme d'une huile visqueuse brune, avec un rendement quantitatif.

Titre (HPLC) : 98.3%

### Références :

1/ Description des conditions d'analyse :

| | |
|---|---|
| méthode HPLC : | |
| Colonne: | Hichrom 100 RP18 5µ (250 x 4.6mm) |
| Débit: | 1ml/min |
| Longueur d'onde: | 210 nm |
| Volume d'injection: | 20µl |
| Eluent : | Acetonitrile 400ml |
| | 0.01M sodium dihydrogen phosphate (pH 2.3) 600ml |
| | Sodium dodecyl sulfate 2.88 g/l. |
| Injection: | 20µl of 0.1mg/ml solution. |

### Temps de rétention :

| | |
|---|---|
| p-anisidine | 12.16 min |
| 2-[(4-methoxyphenylamino)-methylene]-malonic acid diethyl ester | 25.0 min |
| 3-ethoxycarbonyl-6-methoxy-4(1H)-quinolinone | 4.15 min |
| 4-chloro-6-methoxyquinoline-3-ethylcarboxylate | 24.1 min |
| 6-methoxyquinoline-3-ethylcarboxylate | 16.5 min |
| 6-methoxyquinoline-3-carboxamide | 6.0 min |
| 3-amino-6-methoxyquinoline | 16.2 min |
| 3-fluoro-6-methoxyquinoline | 10.0 min |

### Exemple 2 : 3,7-difluoro-6-méthoxy-quinoléine

A une solution aqueuse à 40% d'acide fluoroborique on ajoute sous agitation à une température voisine de -5°C 6,1g de 3-amino-7-fluoro-6-méthoxy-quinoléine, puis on additionne en 20 minutes une solution de 2,6g de nitrite de sodium dans 5,2 cm³ d'eau. Le mélange réactionnel est agité à une température voisine de +3°C pendant 40 minutes, puis filtré. Le solide est lavé par une solution aqueuse à 40% d'acide fluoroborique à -5°C, puis par un mélange d'isopropanol et d'une solution aqueuse à 40% d'acide fluoroborique à -5°C, puis à l'éther éthylique, essoré à sec et séché sous pression réduite. On obtient ainsi 9.95 g d'un solide que l'on dissout dans 80 cm3 de toluène anhydre et cette solution est portée à 92°C pendant 1 heure sous forte agitation. Après refroidissement à température ambiante, on ajoute 50 cm³ de toluène, puis 80 cm³ d'une solution aqueuse saturée en hydrogénocarbonate de sodium. Le milieu réactionnel est décanté, la phase aqueuse est extraite au toluène, les phases organiques sont réunies, lavées par une solution aqueuse saturée en chlorure de sodium, puis séchées sur sulfate de sodium. Après filtration puis évaporation à sec sous pression réduite du toluène, le résidu est chromatographié sur colonne de silice (100g, granulométrie 20-46 µm, éluant : dichlorométhane). Les fractions contenant le produit attendu sont évaporées à sec sous pression réduite. On obtient 2,28g de 3,7-difluoro-6-méthoxy-quinoléine sous la forme d'un solide blanc fondant à 98°C.

### 3-Amino-7-fluoro-6-méthoxy-quinoléine:

A une solution de 133 cm³ de solution aqueuse de soude 2N refroidie à 0°C on ajoute goutte à goutte en 30 minutes 2,4 cm³ de brome, puis 111 cm³ de pyridine. On ajoute ensuite à cette solution, toujours à 0°C, 10, 1 g de 7-fluoro-6-méthoxy-quinoléine-3-carboxamide, et on agite à 0°C pendant 2h30. On laisse ensuite le milieu réactionnel se réchauffer à température ambiante, puis on le chauffe sous agitation à 60°C pendant 18h. On le laisse ensuite se refroidir à température ambiante, puis on ajoute 100 cm³ d'eau, puis 100 cm³ d'acétate d'éthyle. Le milieu réactionnel est décanté, la phase aqueuse est extraite par l'acétate d'éthyle, les phases aqueuses sont réunies, lavées à l'eau puis séchées sur sulfate de sodium et évaporées à sec sous pression réduite. On obtient 8,25g d'un résidu solide qui est trituré dans 150 cm³ d'éther isopropylique et filtré. Le solide est lavé à l'éther isopropylique, puis au pentane. Après séchage on obtient 6,20g de 3-amino-7-fluoro-6-méthoxy-quinoléine sous la forme d'un solide brun clair fondant à 153°C.

### 7-Fluoro-6-méthoxy-quinoléine-3-carboxamide :

A une solution de 13,9g de 4-chloro-7-fluoro-6-méthoxy-quinoléine-3-carboxamide dans 278 cm³ de diméthylformamide on ajoute 5,58g de formiate de sodium et 3,16g de tétrakis(triphénylphosphine) palladium, et on chauffe cette solution sous atmosphère d'argon à 100°C pendant 5 heures. Après refroidissement à température ambiante, le milieu réactionnel est filtré. Le filtrat est concentré sous pression réduite pour obtenir 200cm³ d'une solution à laquelle on ajoute 600 cm³ d'eau. Le précipité formé est filtré, lavé à l'eau puis séché à 50°C sous pression réduite. Le solide obtenu est lavé par du toluène, puis 2 fois par de l'éther éthylique, puis par du pentane. On obtient 10,7g de 7-fluoro-6-méthoxy-quinoléine-3-carboxamide sous la forme d'un solide beige fondant à 231°C.

### 4-Chloro-7-fluoro-6-méthoxy-quinoléine-3-carboxamide :

Une solution de 15,83g d'acide 7-fluoro-4-hydroxy-6-méthoxy-quinoléine-3-carboxylique dans 40 cm³ de chlorure de phosphoryle est portée sous agitation à 100°C pendant 3 heures. Après refroidissement à température ambiante, le milieu réactionnel est distillé sous pression atmosphérique pour éliminer le chlorure de phosphoryle. Le résidu est dissous dans 70 cm³ de dichlorométhane, puis on fait barboter de l'ammoniac dans cette solution maintenue à 25°C sous agitation pendant 5 heures. Le milieu réactionnel est ensuite filtré, le solide obtenu est lavé au dichlorométhane, puis séché à 50°C sous pression réduite. On obtient 14,05g de 4-chloro-7-fluoro-6-méthoxy-quinoléine-3-carboxamide sous la forme d'un solide blanc cassé fondant à 228°C.

### Acide 7-fluoro-4-hydroxy-6-méthoxy-quinoléine-3-carboxylique :

Une solution de 23,57g de 7-fluoro-4-hydroxy-6-méthoxy-quinoléine-3-carboxylate d'éthyle dans 71 cm³ d'une solution aqueuse de soude 5N est portée à 100°C sous agitation pendant 3 heures. Après retour à température ambiante, le milieu réactionnel est acidifié par addition de 32,5 cm³ d'une solution aqueuse d'acide chlorhydrique à 37%. Après addition de 150 cm³ d'eau, le précipité obtenu est filtré et le solide est lavé à l'eau. On obtient après séchage à l'air libre 22g d'acide 7-fluoro-4-hydroxy-6-méthoxy-quinoléine-3-carboxylique sous la forme d'un solide crème fondant à 275°C.

### 7-fluoro-4-hydroxy-6-méthoxy-quinoléine-3-carboxylate d'éthyle :

Une solution de 37,75g de 2-[(3-fluoro-4-méthoxy-phénylamino)-méthylène]-malonate de diéthyle dans 170 cm³ de diphényléther est portée à 245°C sous agitation pendant 3,5 heures. Après retour à température ambiante, on ajoute 220 cm³ de cyclohexane, on filtre le précipité ainsi obtenu et lave du cyclohexane, puis par du pentane et l'essore à sec. On obtient 24,10g de 7-fluoro-4-hydroxy-6-méthoxy-quinoléine-3-carboxylate d'éthyle sous la forme d'un solide fondant à 280°C.

### 2-[(3-fluoro-4-méthoxy-phénylamino)-méthylène]-malonate de diéthyle :

Un mélange de 15,61g de 3-fluoro-4-méthoxy-aniline et de 24,25g d'éthoxy-méthylène malonate de diéthyle est porté à 100°C sous agitation pendant 2,5 heures. Après refroidissement à température ambiante puis évaporation à sec à 50°C sous pression réduite, on obtient 35g de 2-[(3-fluoro-4-méthoxy-phénylamino)-méthylène]-malonate de diéthyle sous la forme d'un solide beige fondant à 63°C.

### Exemple 3 : 3,8-difluoro-6-méthoxy-quinoléine

En opérant comme décrit à l'exemple 2, mais à partir de 2,35g de 3-amino-8-fluoro-6-méthoxy-quinoléine on obtient 1,35g de 3,8-difluoro-6-méthoxy-quinoléine sous la forme d'un solide blanc fondant à 122°C

Caractéristiques des intermédiaires de synthèse :
3-Amino-8-fluoro-6-méthoxy-quinoléine : solide marron fondant à 135°C.
8-Fluoro-6-méthoxy-quinoléine-3-carboxamide : solide beige fondant à 248°C.
4-Chloro-8-fluoro-6-méthoxy-quinoléine-3-carboxamide : solide marron clair fondant à 220°C.
Acide 8-fluoro-4-hydroxy-6-méthoxy-quinoléine-3-carboxylique : solide beige fondant vers 280°C.
8-Fluoro-4-hydroxy-6-méthoxy-quinoléine-3-carboxylate d'éthyle : solide brun clair fondant à 221°C.
2-[(2-fluoro-4-méthoxy-phénylamino)-méthylène]-malonate de diéthyle : spectre de masse IE m/z= 311 (M⁺).

On utilise au départ la 2-fluoro 4-méthoxyamiline.

### Exemple 4 : 3,6-difluoroquinoléine

En opérant comme décrit à l'exemple 2, mais à partir de 3-amino-6-fluoro-quinoléine on obtient la 3,6-difluoroquinoléine , spectre de masse IE m/z=165 (M⁺).

Caractéristiques des intermédiaires de synthèse :
3-Amino-6-fluoro-quinoléine : spectre de masse IE m/z=162 (M⁺).
6-Fluoro-quinoléine-3-carboxamide : spectre de masse IE m/z=190 (M⁺).
4-Chloro-6-fluoro-quinoléine-3-carboxamide : spectre de masse IE m/z=224 (M⁺).
Acide 4-hydroxy-6-fluoro-quinoléine-3-carboxylique : spectre de masse IE m/z=207 (M⁺).
4-Hydroxy-6-fluoro-quinoléine-3-carboxylate d'éthyle : spectre de masse IE m/z=235 (M⁺).
2-[(2-Fluoro-4-méthoxy-phénylamino)-méthylène]-malonate de diéthyle : spectre de masse IE m/z= 281 (M⁺).

On utilise au départ la 4-fluoroamiline.

## Revendications

1. Procédé de préparation des quinoléines 3-fluorées de formule générale (I) : 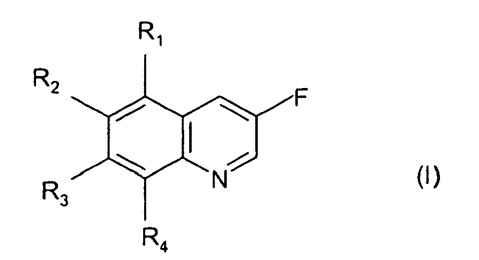 dans laquelle R₁, R₂, R₃ et R₄, identiques ou différents, représentent :
- un atome d'hydrogène ou de fluor ;
- un radical alkyle linéaire, ramifié ou cyclique, éventuellement substitué par un à trois atomes de fluor, par un groupement OR₅ dans lequel R₅ représente un radical alkyle linéaire ou ramifié, un atome d'hydrogène ou un groupement protecteur de radical hydroxy, ou par un groupement NR'R" dans lequel R' et R", indentiques ou différents, représentent un radical alkyle linéaire ou ramifié, un atome d'hydrogène ou un groupement protecteur du radical amino ;
- un groupement OR₆ dans lequel R₆ représente un atome d'hydrogène, un groupement protecteur du phénol ou un radical alkyle linéaire ou ramifié, éventuellement substitué par un à trois atomes de fluor, par OR₅ ou par NR'R" tels que définis ci-dessus ;
- un groupement NR'₁R"₁ dans lequel R'₁ et R"₁ ont les valeurs de R' et R" ou représentent un radical alkyle linéaire ou ramifié substitué par un à trois atomes de fluor, par un groupement OR₅ tel que défini ci-dessus ou par un groupement NR'R" tel que défini ci-dessus ;
un groupement CO₂Rₐ, dans lequel Rₐ représente un atome d'hydrogène, un radical alkyle linéaire ou ramifié ou un groupement protecteur du radical carboxy ;
- un radical phényle ou un radical hétéroaryle, éventuellement substitué par un ou plusieurs des substituants mentionnés plus haut,
**caractérisé en ce que** l'on soumet un composé de formule générale (IV) : 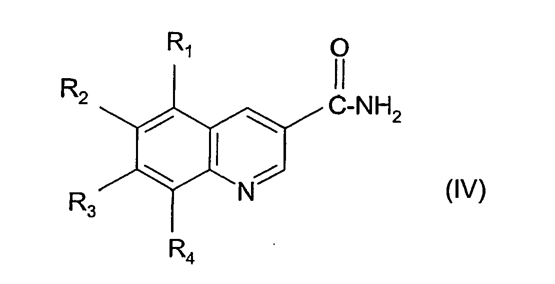 dans laquelle R₁, R₂, R₃ et R₄ conservent les définitions précitées, à une dégradation d'Hofmann, pour obtenir un composé de formule générale (III) 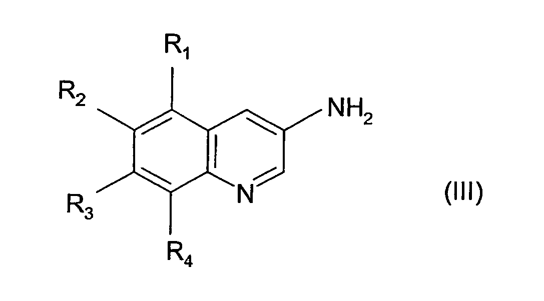 dans laquelle R₁, R₂, R₃ et R₄ conservent les définitions précitées, que l'on traite dans des conditions susceptibles de former le sel de diazonium de formule générale (II) 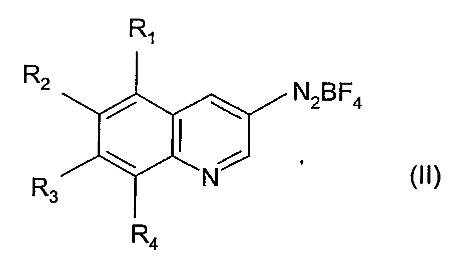 dans laquelle R₁, R₂, R₃ et R₄ conservent les définitions précitées, que l'on chauffe au sein d'un solvant organique inerte.

2. Procédé selon la revendication 1, **caractérisé en ce que** l'on prépare le sel de diazonium par action d'un sel alcalin ou d'un ester d'alkyle de l'acide nitreux et d'acide fluoroborique ou du complexe trifluorure de bore-éther éthylique.

3. Procédé selon la revendication 1, **caractérisé en ce que** le composé de formule générale (IV) est obtenu en soumettant un composé de formule générale (V) : 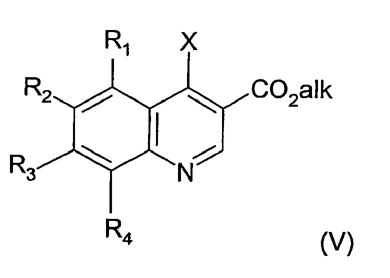 dans laquelle R₁, R₂, R₃ et R₄ sont définis comme à la revendication 1, X représente un atome de chlore ou de brome, et alk représente un radical alkyle renfermant de 1 à 6 atomes de carbone, à l'action d'un agent d'hydrogénolyse, puis à celle de l'ammoniaque pour obtenir successivement le composé de formule (VI) 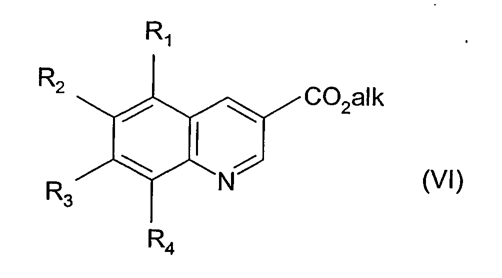 que l'on isole ou non, puis le composé de formule (IV) ;

4. Procédé selon la revendication 1, **caractérisé en ce que** le composé de formule générale (IV) est obtenu en soumettant un composé de formule générale (V) telle que définie à la revendication 3, à l'action de l'ammoniaque, pour obtenir un composé de formule générale (VII) : 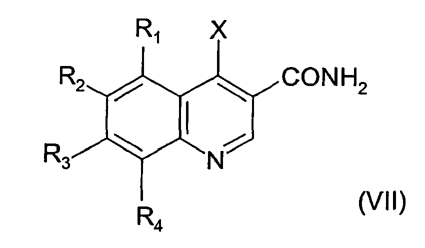 dans laquelle R₁, R₂, R₃, R₄ et X sont définis comme à la revendication 1, que l'on soumet à l'action d'un agent d'hydrogénolyse pour obtenir le composé attendu.

5. Procédé selon la revendication 3 ou 4, **caractérisé en ce que** le composé de formule générale (V) est obtenu en soumettant un composé de formule générale (VIII) : 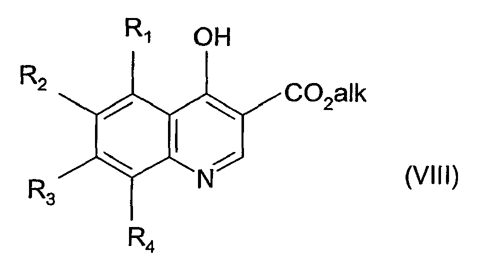 dans laquelle R₁, R₂, R₃, R₄ et alk sont définis comme à la revendication 1, à l'action de l'oxychlorure ou de l'oxybromure de phosphore.

6. Procédé selon la revendication 1, **caractérisé en ce que** le composé de formule générale (IV) est obtenu en traitant un composé de formule générale (VIII), telle que définie à la revendication 5, par une base, pour obtenir un acide correspondant de formule générale (IX) : 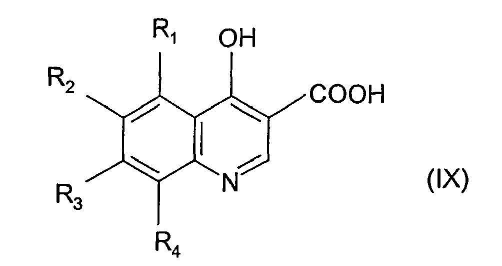 dans laquelle R₁, R₂, R₃ et R₄ sont définis comme à la revendication 1, que l'on soumet à l'action de l'oxychlorure ou de l'oxybromure de phosphore, pour obtenir un composé de formule générale (X) : 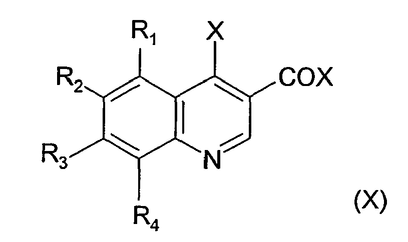 dans laquelle R₁, R₂, R₃ et R₄ conservent les définitions précitées et X est défini comme à la revendication 3, lequel est soumis à l'action de l'ammoniac, pour obtenir un composé de formule générale (VII) telle que définie à la revendication 4, que l'on soumet à l'action d'un agent d'hydrogénolyse.

7. Procédé selon la revendication 1 ou 6, **caractérisé en ce que** l'on utilise au départ un composé de formule générale (IV) ou (IX) dans laquelle les éventuels substituants sensibles sont protégés.

8. Procédé selon l'une quelconque des revendications 1 à 7, **caractérisé en ce que** l'on met en oeuvre des composés dans lesquels R₁, R₂, R₃ et R₄, identiques ou différents, représentent un atome d'hydrogène, un atome de fluor, un radical alkyle linéaire ou ramifié éventuellement substitué tel que défini à la revendication 1, ou un radical OR₆ tel que défini à la revendication 1.

9. Procédé selon la revendication 8, **caractérisé en ce que** l'on met en oeuvre des composés dans lesquels R₁, R₂, R₃ et R₄ identiques ou différents, représentent un atome d'hydrogène, un atome de fluor ou un radical alkoxy linéaire ou ramifié, renfermant de 1 à 10 atomes de carbone.

10. Les composés de formule générale (II) telle que définie à la revendication 1.

## Claims

1. A process for preparing 3-fluoroquinolines of formula (I): 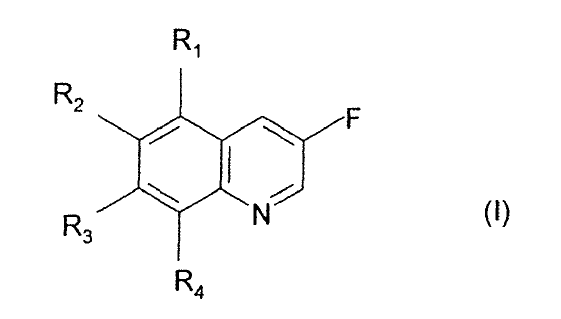 **characterized in that**
R₁, R₂, R₃ and R₄, which may be identical or different, represent:
- a hydrogen or fluorine atom;
- a linear, branched or cyclic alkyl radical optionally substituted with one to three fluorine atoms, with a group OR₅ in which R₅ represents a linear or branched alkyl radical, a hydrogen atom or a hydroxyl radical-protecting group, or with a group NR'R" in which R' and R", which may identical or different, represent a linear or branched alkyl radical, a hydrogen atom or an amino radical-protecting group;
- a group OR₆ in which R₆ represents a hydrogen atom, a phenol-protecting group or a linear or branched alkyl radical optionally substituted with one to three fluorine atoms, with OR₅ or with NR'R" as defined above;
- a group NR'₁R"₁ in which R'₁ and R"₁ have the values of R' and R" or represent a linear or branched alkyl radical substituted with one to three fluorine atoms, with a group OR₅ as defined above or with a group NR'R" as defined above;
- a group CO₂Rₐ, in which Rₐ represents a hydrogen atom, a linear or branched alkyl radical or a carboxyl radical-protecting group;
- a phenyl radical or a heteroaryl radical, optionally substituted with one or more of the substituents mentioned above,
wherein:
a compound of formula (IV): 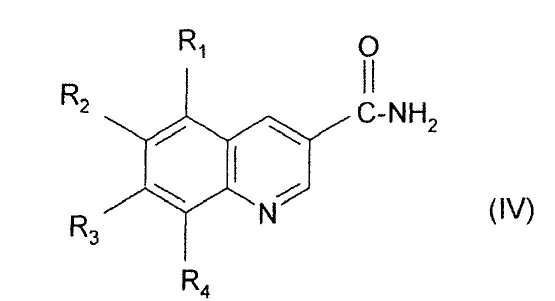
in which R₁, R₂, R₃ and R₄ are as defined above, is subjected to a Hofmann degradation, so as to obtain a compound of general formula (III): 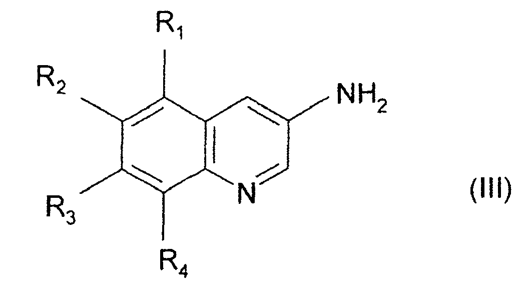 in which R₁, R₂, R₃ and R₄ are as defined above, which is treated under conditions capable of forming the diazonium salt of general formula (II): 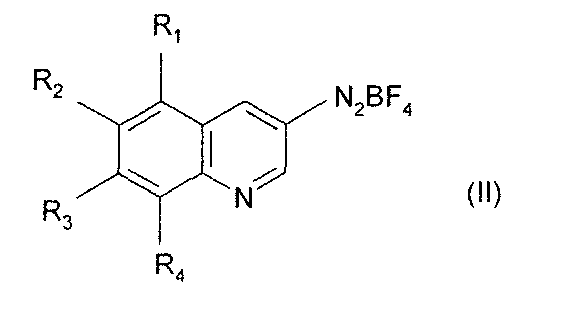 in which R₁, R₂, R₃ and R₄ are as defined above,
which is heated in an inert organic solvent

2. The process as set forth in claim 1, **characterized in that** the diazonium salt is prepared by the action of an alkali metal salt or of an alkyl ester of nitrous acid and of fluoroboric acid or of the boron trifluoride-ethyl ether complex.

3. The process as set forth in claim 1, **characterized in that** the compound of formula (IV) is obtained by subjecting a compound of formula (V): 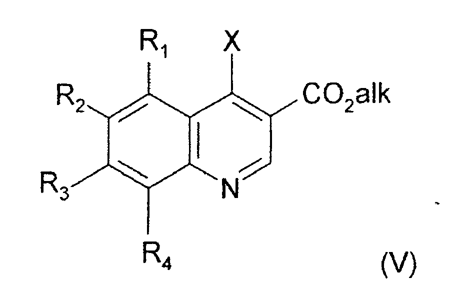 in which R₁, R₂, R₃ and R₄ are defined as in claim 1, X represents a chlorine atom or a bromine atom and alk represents an alkyl radical containing from 1 to 6 carbon atoms, to the action of a hydrogenolysis agent, and then to aqueous ammonia, so as to obtain successively the compound of formula (VI): 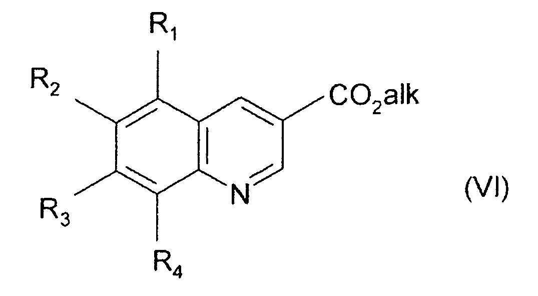 which may or may not be isolated, and then the compound of formula (IV).

4. The process as set forth in claim 1, **characterized in that** the compound of formula (IV) is obtained by subjecting a compound of formula (V) as defined in claim 3,
to the action of aqueous ammonia, so as to obtain a compound of formula (VII): 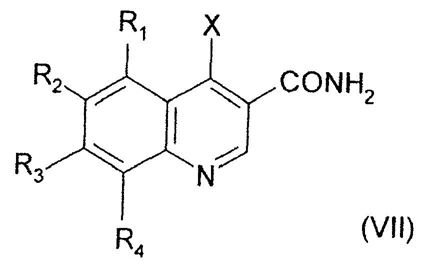 in which R₁, R₂, R₃, R₄ and X are as defined in claim 1, which is subjected to the action of a hydrogenolysis agent so as to obtain the expected compound.

5. The process as set forth in claim 3 or 4, **characterized in that** the compound of formula (V) is obtained by subjecting a compound of formula (VIII): 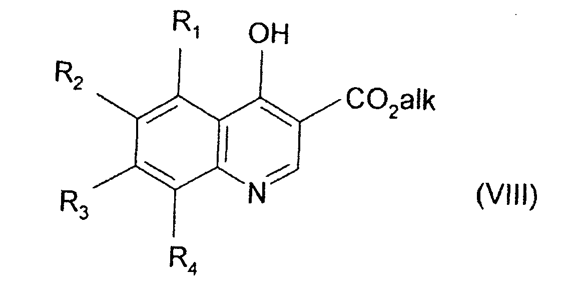 wherein R₁, R₂, R₃, R₄ and alk are as defined in claim 1, to the action of phosphorus oxychloride or phosphorus oxybromide.

6. The process as set forth in claim 1, **characterized in that** the compound of formula (IV) is obtained by treating a compound of formula (VIII), as defined in claim 5, with a base, so as to obtain a corresponding acid of formula (IX): 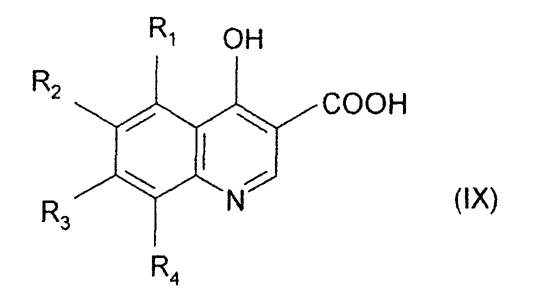 in which R₁, R₂, R₃ and R₄ are as defined in claim 1, which is subjected to the action of phosphorus oxychloride or of phosphorus oxybromide, so as to obtain a compound of formula (X) : 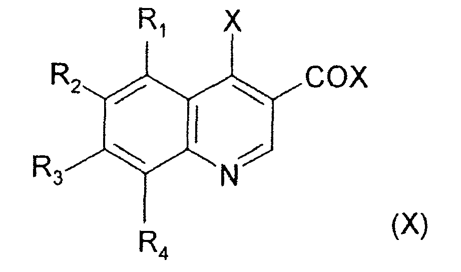 in which R₁, R₂, R₃ and R₄ retain the above mentioned definitions and X is defined as in claim 3, which is subjected to the action of ammonia so as to obtain a compound of formula (VII) as defined in claim 4, which is subjected to the action of a hydrogenolysis agent.

7. The process as set forth in claim 1 or 6, **characterized in that** use is made at the start of a compound of formula (IV) or (IX) in which the possible sensitive substituents are protected.

8. The process as set forth in any one of claims 1 to 7, **characterized in that** use is made of compounds in which R₁, R₂, R₃ and R₄, which may be identical or different, represent a hydrogen atom, a fluorine atom, an optionally substituted linear or branched alkyl radical as defined in claim 1, or a radical OR₆ as defined in claim 1.

9. The process as set forth in claim 8, **characterized in that** use is made of compounds in which R₁, R₂, R₃ and R₄, which may be identical or different, represent a hydrogen atom, a fluorine atom or a linear or branched alkoxy radical containing from 1 to 10 carbon atoms.

10. A compound of formula (II) as defined in claim 1.

## Patentansprüche

1. Verfahren zur Herstellung von 3-Fluorchinolinen der allgemeinen Formel (I): 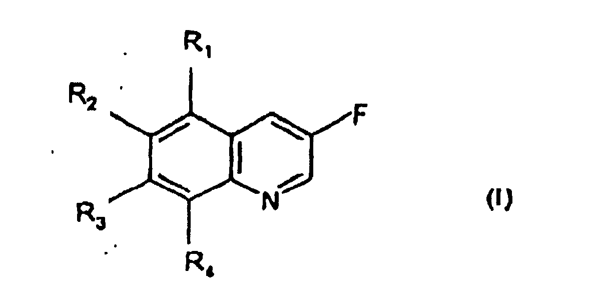 worin R₁, R₂, R₃ und R₄, gleich oder verschieden, Folgendes darstellen:
- ein Wasserstoff- oder Fluoratom;
- einen linearen, verzweigten oder cyclischen Alkylrest, der gegebenenfalls durch ein bis drei Fluoratome, durch eine OR₅-Gruppe, wobei R₅ einen linearen oder verzweigten Alkylrest ein Wasserstoffatom oder eine Schutzgruppe des Hydroxyrests darstellt, oder durch eine NR'R''-Gruppe substituiert ist, wobei R' und R'', gleich oder verschieden, einen linearen oder verzweigten Alkylrest ein Wasserstoffatom oder eine Schutzgruppe des Aminorests darstellen;
- eine OR₆-Gruppe, wobei R₆ ein Wasserstoffatom eine Schutzgruppe des Phenols oder einen linearen oder verzweigten Alkylrest darstellt,, der gegebenenfalls durch ein bis drei Fluoratome, durch OR₅ oder durch NR'R'', wie oben definiert, substituiert ist;
- eine NR'₁R''₁-Gruppe, wobei R'₁ und R''₁ die Werte von R' und R'' haben oder einen linearen oder verzweigten Alkylrest darstellen, der durch ein bis drei Fluoratome, durch eine OR₅-Gruppe, wie oben definiert, oder durch eine NR'R''-Gruppe, wie oben definiert, substituiert ist;
- eine CO₂Rₐ-Gruppe, wobei Rₐ ein Wasserstoffatom, einen linearen oder verzweigten Alkylrest oder eine Schutzgruppe des Carboxyradicals darstellt;
- ein Phenylrest oder ein Heteroarylrest, der gegebenenfalls durch einen oder mehrere der weiter oben erwähnten Substituenten substituiert ist,
**dadurch gekennzeichnet, dass** eine Verbindung der folgenden allgemeinen Formel (IV): 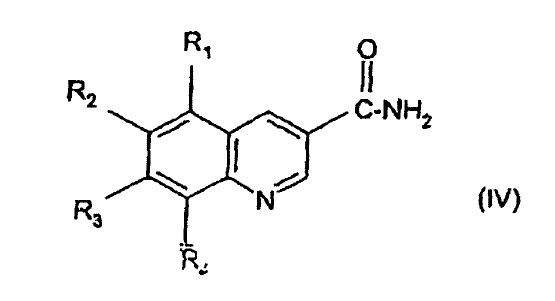 worin R₁, R₂, R₃ und R₄ die vorerwähnten Definitionen beibehalten, für einen Hofmann-Abbau erwärmt wird, um eine Verbindung der folgenden allgemeinen Formel (III) zu erhalten: 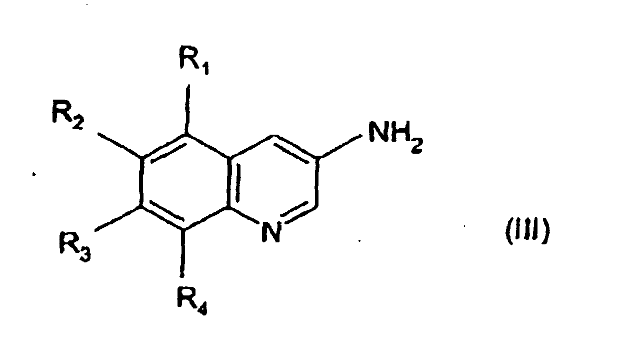 worin R₁, R₂, R₃ und R₄ die vorerwähnten Definitionen beibehalten, die unter Bedingungen behandelt wird, bei denen das Diazoniumsalz der folgenden allgemeinen Formel (II) gebildet werden kann: 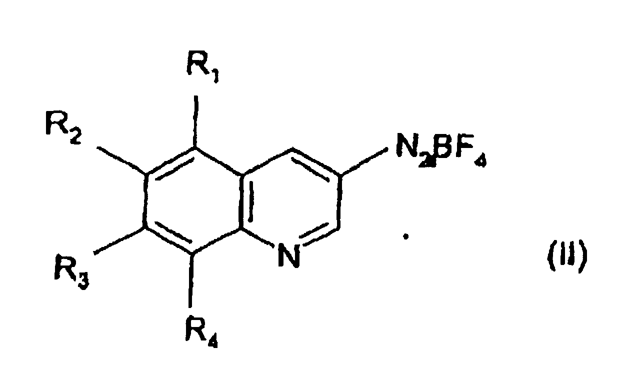 worin R₁, R₂, R₃ und R₄ die vorerwähnten Definitionen beibehalten, das in einem inerten organischen Lösemittel erwärmt wird.

2. Verfahren nach Anspruch 1, **dadurch gekennzeichnet, dass** das Diazoniumsalz durch Wirkung eines Alkalisalzes oder eines Alkylesters der salpetrigen Säure und Fluoroborsäure oder in Gegenwart des Bortrifluorid-Ethylether-Komplexes hergestellt wird.

3. Verfahren nach Anspruch 1, **dadurch gekennzeichnet, dass** die Verbindung der allgemeinen Formel (IV) erhalten wird, indem ein Verbindung der folgenden allgemeinen Formel (V): 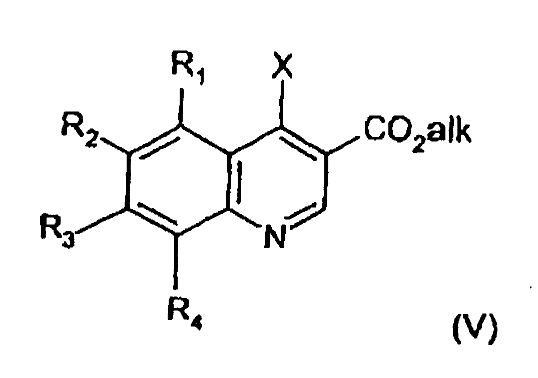 worin R₁, R₂, R₃ et R₄ definiert sind, wie in Anspruch 1, X ein Chlor- oder Boratom darstellt, und alk einen Alkylrest darstellt, der 1 bis 6 Kohlenstoffatome beinhaltet, der Wirkung eines Hydrogenolysemittels, dann der von Ammoniak unterzieht, um nacheinander die folgende Verbindung der Formel (VI): 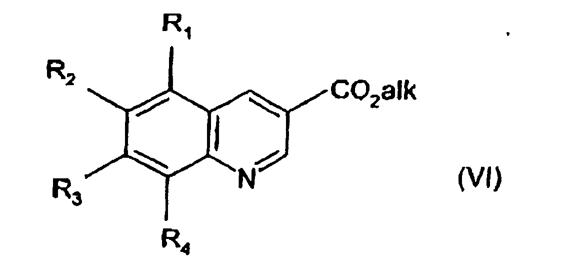 die isoliert wird oder nicht, dann die Verbindung der Formel (IV) zu erhalten.

4. Verfahren nach Anspruch 1, **dadurch gekennzeichnet, dass** die Verbindung der allgemeinen Formel (IV) erhalten wird, indem eine Verbindung der allgemeinen Formel (V), wie in Anspruch 3 definiert, der Wirkung von Ammoniak unterzogen wird, um eine Verbindung der folgenden allgemeinen Formel (VII) zu erhalten: 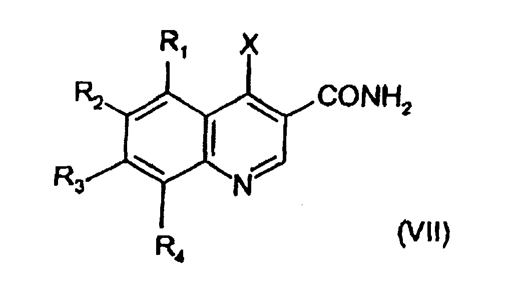 worin R₁, R₂, R₃, R₄ und X definiert sind, wie in Anspruch 1, die der Wirkung eines Hydrogenolysemittels unterzogen wird, um die erwartete Verbindung zu erhalten.

5. Verfahren nach Anspruch 3 oder 4, **dadurch gekennzeichnet, dass** die Verbindung der allgemeinen Formel (V) erhalten wird, indem eine Verbindung der folgenden allgemeinen Formel (VIII): 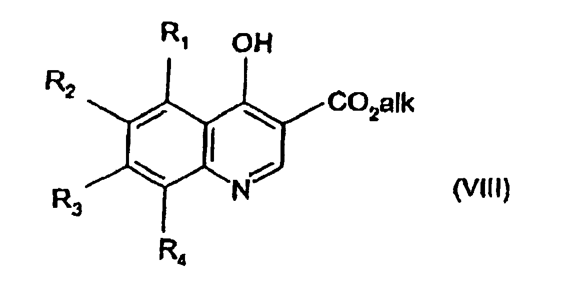 worin R₁, R₂, R₃, R₄ und alk definiert sind, wie in Anspruch 1, der Wirkung von Phosphoroxidchlorid oder Phosphoroxidbromid unterzogen wird.

6. Verfahren nach Anspruch 1, **dadurch gekennzeichnet, dass** die Verbindung der allgemeinen Formel (IV) erhalten wird, indem eine Verbindung der allgemeinen Formel (VIII), wie in Anspruch 5 definiert, mit einer Base behandelt wird, um eine Säure zu erhalten, die der folgenden allgemeinen Formel (IX) entspricht: 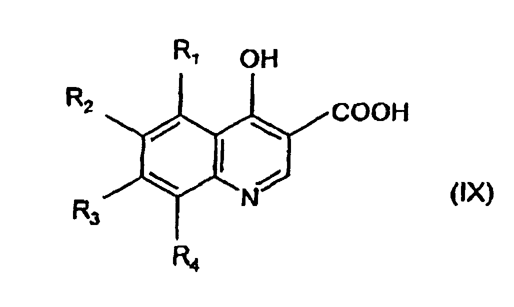 worin R₁, R₂, R₃ und R₄ definiert sind, wie in Anspruch 1, die der Wirkung von Phosphoroxidchlorid oder Phosphoroxidbromid unterzogen wird, um die Verbindung der folgenden allgemeinen Formel (X) zu erhalten. 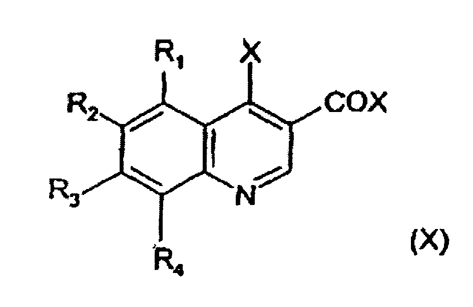 worin R₁, R₂, R₃ und R₄ die vorerwähnten Definitionen beibehalten und X wie in Anspruch 3 definiert ist, die der Wirkung von Ammoniak unterzogen wird, um eine Verbindung der allgemeinen Formel (VII) zu erhalten, wie in Anspruch 4 definiert, die der Wirkung eines Hydrogenolysemittels unterzogen wird.

7. Verfahren nach Anspruch 1 oder 6, **dadurch gekennzeichnet, dass** zu Beginn eine Verbindung der allgemeinen Formel (IV) oder (IX) verwendet wird, worin die möglicherweise vorhandenen empfindlichen Substituenten geschützt sind.

8. Verfahren nach irgendeinem der Ansprüche 1 bis 7, **dadurch gekennzeichnet, dass** Verbindungen eingesetzt werden, worin R₁, R₂, R₃ und R₄, gleich oder verschieden, ein Wasserstoffatom, ein Fluoratom, einen linearen oder verzweigten Alkylrest, der gegebenenfalls substituiert ist, wie in Anspruch 1 definiert, oder einen OR₆-Rest, wie in Anspruch 1 definiert, darstellen.

9. Verfahren nach Anspruch 8, **dadurch gekennzeichnet, dass** Verbindungen eingesetzt werden, worin R₁, R₂, R₃ und R₄, gleich oder verschieden, ein Wasserstoffatom, ein Fluoratom oder einen linearen oder verzweigten Alkoxyrest darstellen, der 1 bis 10 Kohlenstoffatome beinhaltet.

10. Verbindungen der allgemeinen Formel (II), wie in Anspruch 1 definiert.
